# EUROPÄISCHE PATENTSCHRIFT

(11) **EP 3 010 924 B1**
(45) Veröffentlichungstag und Bekanntmachung des Hinweises auf die Patenterteilung: **14.09.2016**
(21) Anmeldenummer: 14736289.1
(22) Anmeldetag: 12.06.2014
(51) Int. Cl.: C07D 493/04

(54) **UV-HÄRTUNGSKOMPATIBLE PHOTOCHROME ANNELLIERTE NAPHTHOPYRANE**
PHOTOCHROMIC FUSED NAPHTHOPYRANES COMPATIBLE TO UV-CURING
NAPHTOPYRANES FUSIONNES PHOTOCHROMES COMPATIBLES AU DURCISSEMENT PAR RAYONS UV

(30) Priorität: 17.06.2013 DE 102013010032; 26.11.2013 DE 102013019706
(43) Veröffentlichungstag der Anmeldung: 27.04.2016
(73) Patentinhaber: Rodenstock GmbH, 80687 München (DE)
(72) Erfinder: WEIGAND, Udo, 81247 München (DE); ZINNER, Herbert, 85296 Rohrbach (DE); ROHLFING, Yven, 81547 München (DE)
(74) Vertreter: Müller-Boré & Partner Patentanwälte PartG mbB
(86) Internationale Anmeldenummer: PCT/EP2014/001602
(87) Internationale Veröffentlichungsnummer: WO 2014/202194

(56) Entgegenhaltungen:
- EP-A1- 1 978 022
- EP-A1- 2 305 768
- US-B1- 6 506 538

## Beschreibung

Die vorliegende Erfindung betrifft UV-härtungskompatible photochrome einfachsowie zweifach-annellierte Naphthopyrane gemäß den allgemeinen Formeln (I) oder (II) und deren Verwendung in Kunststoffen aller Art, insbesondere für ophthalmische Zwecke. Die erfindungsgemäßen photochromen Verbindungen zeichnen sich durch ihre hervorragende UV-Härtungskompatibilität aus, d.h. sie überstehen - eingebracht z.B. in eine Acrylatmonomer-Matrix mit UV-Initiator-unbeschadet eine durch starkes UV-Licht ausgelöste radikalische Polymerisation der Matrix. Die erfindungsgemäßen photochromen Farbstoffe zeichnen sich darüber hinaus bei geeigneter Substituentenwahl durch zwei ausgeprägte Absorptionsbanden der offenen Form im sichtbaren Wellenlängenbereich aus, d.h. mit einem derartigen Molekül lassen sich zwei konventionelle photochrome Farbstoffe - mit jeweils einer diskreten Absorptionsbande - ersetzen. Darüber hinaus weisen sie bei sehr hoher Leistung eine sehr gute Lebensdauer aus. Sie sind in Kunststoffen aller Art einsetzbar.

Seit langem sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, dass diese Farbstoffmoleküle durch Lichtenergie in einen angeregten Zustand ("offene Form") übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho[1,2-b]pyrane oder die 3,3-Diaryl-3H-naphtho[2,1-b]pyrane, deren offene, angeregte Formen verschiedene Eindunkelungsfarben von Gelb bis Rotviolett zeigen.

Wird diese Verbrückung nur über ein Atom hergestellt, so ergibt sich ein an das Naphthopyran annellierter Fünfring. Beispiele finden sich in US 5,645,767, US 5,723,072 sowie US 5,955,520 für ein C-Atom ("Indeno-Annellierung") und in US 6,018,059 für ein O-Atom.

In US 5,723,072 kann zusätzlich an diesem Grundsystem ein un-, mono- oder disubstituierter heterocyclischer Ring an der g-, h-, i-, n-, o- oder p-Seite des Indenonaphthopyrans annelliert sein. Es werden demzufolge Indeno[2,1-f]naphtho[1,2-b]pyrane mit einer sehr großen Variationsbreite an möglichen Substituenten offenbart.

Auch WO 96/14596, WO 99/15518, US 5,645,767, WO 98/32037 und US 5,698,141 beschreiben vom 2H-Naphtho[1,2-b]pyran abgeleitete photochrome indenoannellierte Naphthopyran-Farbstoffe, die sie enthaltenden Zusammensetzungen sowie ein Verfahren zu deren Herstellung. In US 5,698,141 kann zusätzlich an diesem Grundsystem ein un-, mono- oder disubstituierter heterozyklischer Ring an der g-, h-, i-, n-, o- oder p-Seite des Indenonaphthopyrans annelliert sein. Von der jeweils sehr umfangreichen Substituentenliste sind auch ganz spezielle Spiro-Verbindungen umfaßt, und zwar solche Systeme mit einer spiroheterozyklischen Gruppe, worin einschließlich des Spiroatoms an der 13-Position des Grundsystems ein 5- bis 8-gliedriger Ring, der stets zwei Sauerstoffatome enthält, vorhanden ist. Eine weitere Auslegung des Spiroringes findet sich in JP 344762/2000.

Wird diese Verbrückung über zwei Atome erzeugt, so ergibt sich ein annellierter Sechsring mit allein für C, O und N vielfältigen Möglichkeiten. Verbindungen mit einer Lactam-Brücke werden in US 6,379,591 beschrieben, Verbindungen mit einer unsubstituierten CH₂-CH₂-Brücke sowie einem weiteren annellierten Heterocyclus in US 6,426,023. US 6,506,538 beschreibt die carbocyclischen Analogverbindungen, bei denen die H-Atome in der Brücke durch OH, (C₁ - C₆)-Alkyl oder (C₁ - C₆)-Alkoxy oder zwei H-Atome an einem C-Atom durch =O ersetzt sein können. US 6,022,495 beschreibt u.v.a. Verbindungen mit einer O-CR¹R²-Brücke, ebenso WO 2009/024271 und WO 2013/045086.

Wird diese Verbindung durch drei Atome erzeugt, so ergibt sich ein annellierter Siebenring mit vielen Variationsmöglichkeien durch Einfügen von Heteroatomen. Verbindungen mit einer CH₂-CH₂-CH₂-Brücke sind in US 6,558, 583 beschrieben. Auch hier können die H-Atome in der Brücke durch OH, (C₁ - C₆)-Alkyl oder (C₁ - C₆)-Alkoxy oder zwei H-Atome an einem C-Atom durch =O ersetzt sein. Sie absorbieren bei gleichem Substitutionsmuster kürzerwellig als die annellierten Sechsringe.

Die verschiedenen, im Stand der Technik verfügbaren photochromen Farbstoffe haben jedoch Nachteile, die bei der Verwendung in Sonnenschutzgläsern den Tragekomfort des Brillenträgers wesentlich beeinträchtigen. Zum einen weisen die Farbstoffe eine nicht ausreichend langwellige Absorption im angeregten wie im nicht angeregten Zustand auf. Zum anderen liegt häufig eine zu hohe Temperaturempfindlichkeit der Eindunkelung vor, wobei gleichzeitig eine zu langsame Aufhellung eintreten kann. Darüber hinaus besitzen die im Stand der Technik verfügbaren Farbstoffe oft eine ungenügende Lebensdauer und erlauben damit nur eine geringe Haltbarkeit der Sonnenschutzgläser. Letzteres macht sich in schnell nachlassender Leistung und/oder starker Vergilbung bemerkbar.

Den vorstehenden, im Stand der Technik verfügbaren photochromen Farbstoffen ist gemeinsam, dass sie nur eine Absorptionsbande der offenen Form im sichtbaren Wellenlängenbereich zeigen. Um in Neutralfarben - d.h. in Grau- oder Brauntönen - eindunkelnde phototrope Gläser zu realisieren, ist insofern ein Abstimmungsprozeß zwischen den verschiedenen photochromen Farbstoffen einer Mischung hinsichtlich Aufhellungsgeschwindigkeit, Lebensdauer sowie spektralen Anregungseigenschaften erforderlich, damit das phototrope Glas zu jedem Zeitpunkt des Eindunklungs- und Aufhellungscylus denselben Farbton aufweist. Es wäre daher äußerst wünschenswert, auf diesen Abstimmungsprozeß verzichten zu können.

Schließlich zeigen viele im Stand der Technik offenbarten photochromen Verbindungen eine mangelnde UV-Härtungskompatibilität, d.h. sie überstehen - eingebracht z.B. in eine Acrylatmonomer-Matrix mit UV-Initiator - nicht unbeschadet eine durch starkes UV-Licht ausgelöste radikalische Polymerisation der Matrix.

Der vorliegenden Erfindung liegt daher die Aufgabe zugrunde, neue photochrome Farbstoffe bereitzustellen, mit denen es möglich ist, in Neutralfarben - d.h. in Grau- oder Brauntönen - eindunkelnde phototrope Gläser mit nur einem solchen photochromen Farbstoff zu realisieren, wobei sich die Farbstoffe auch durch eine hervorragende UV-Härtungskompatibilität auszeichnen sollen. Solche photochrome Farbstoffe sollen sich zudem durch die Kombination von langwelligem Absorptionsmaximum der geschlossenen Form mit steiler Kante zum sichtbaren Wellenlängenbereich, hoher Eindunkelungsleistung, sehr schneller Aufhellreaktion und sehr guter Lichtbeständigkeit auszeichnen.

Diese Aufgabe wird durch die in den Ansprüchen gekennzeichneten Ausführungsformen gelöst.

Insbesondere werden photochrome annellierte Naphthopyrane gemäß den allgemeinen Formeln (I) oder (II) bereitgestellt: 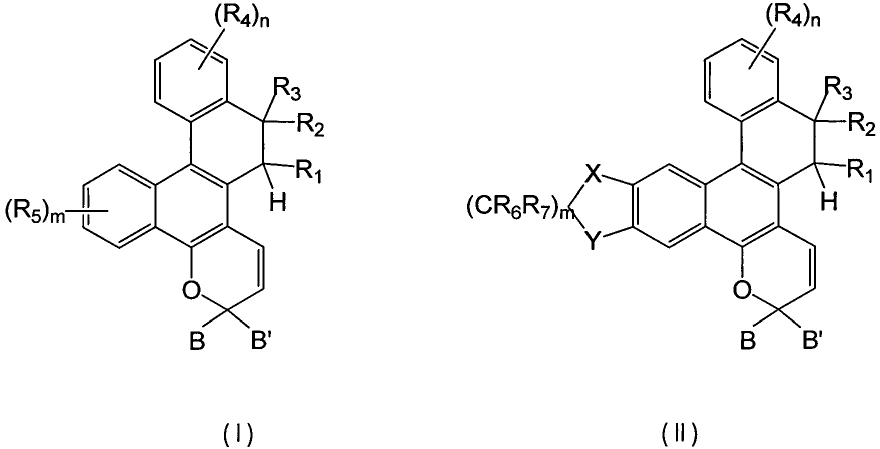
worin die Reste R₃, R₄ und R₅ jeweils unabhängig voneinander einen Substituenten darstellen, ausgewählt aus der Gruppe α, bestehend aus einem Wasserstoffatom, einem (C₁ - C₆)-Alkyl-Rest, einem (C₁ - C₆)-Thioalkyl-Rest, einem (C₃ - C₇)-Cycloalkyl-Rest, der ein oder mehrere Heteroatome, wie beispielsweise O oder S, aufweisen kann, einem (C₁ - C₆)-Alkoxy-Rest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxy-Rest, wobei die Substituenten wiederum aus der Gruppe α ausgewählt sein können; wobei n, wenn nicht 0, eine ganze Zahl von 1 bis 4 darstellt; oder zwei Reste R₄ bilden einen annellierten Benzol-Ring, der un-, mono- oder disubstituiert sein kann, wobei die Substituenten wiederum aus der Gruppe α ausgewählt sein können; oder zwei Reste R₅ bilden einen annellierten Benzol-Ring, der un-, mono- oder disubstituiert sein kann, wobei die Substituenten wiederum aus der Gruppe α ausgewählt sein können;
und worin die Reste R₁ und R₂ jeweils unabhängig voneinander einen (C₁ - C₆)-Alkyl-Rest oder einen un-, mono- oder disubstituierten Phenyl-Rest darstellen, wobei die Substituenten wiederum aus der Gruppe α ausgewählt sein können; und mit der Maßgabe, dass zumindest einer der Reste R₁ oder R₂ einen un-, mono- oder disubstituierten Phenyl-Rest darstellt;
und worin X und Y unabhängig voneinander ausgewählt sind aus der Gruppe, bestehend aus -O-, -S- -N(C₁-C₆)-Alkyl, -NC₆H₅-, -CH₂-, -C(CH₃)₂- oder -C(C₆H₅)₂-;
und worin die Reste R₆ und R₇ jeweils unabhängig voneinander einen Substituenten darstellen, ausgewählt aus der Gruppe α; vorzugsweise ein Wasserstoffatom, einen (C₁ - C₆)-Alkyl-Rest, einen (C₃ - C₇)-Cycloalkyl-Rest oder einen Phenylrest darstellen;
m, wenn nicht 0, eine ganze Zahl von 1 bis 4 darstellt, vorzugsweise 1 oder 2, oder zwei oder mehrere benachbarte -CR₆R₇-Gruppierungen sind Teil eines annellierten Benzol-Ringes, der un-, mono- oder disubstituiert sein kann, wobei die Substituenten wiederum aus der Gruppe α ausgewählt sein können;
oder X und/oder Y stellt zusammen mit der jeweils benachbarten -CR₆R₇-Gruppierung einen annellierten Benzol-Ring dar, der un-, mono- oder disubstituiert sein kann, wobei die Substituenten wiederum aus der Gruppe α ausgewählt sein können;
und worin B und B' unabhängig voneinander aus einer der folgenden Gruppen a) oder b) ausgewählt ist, wobei
   a) ein mono-, di- und trisubstituierter Aryl-Rest ist, wobei der Aryl-Rest Phenyl, Naphthyl oder Phenanthryl ist und
   b) ein un-, mono- und disubstituierter Heteroaryl-Rest ist, wobei der Heteroaryl-Rest Pyridyl, Furanyl, Thienyl, Benzofuranyl, Benzothienyl, 1,2,3,4-Tetrahydrocarbazolyl und Julolidinyl ist;
   wobei die Substituenten der Aryl- und Heteroaryl-Reste in a) und b) ausgewählt sind aus der Gruppe α oder der Gruppe λ, bestehend aus Amino, Mono-(C₁ - C₆)-alkylamino, Di-(C₁ - C₆)-alkylamino, am Phenylring un-, mono- oder disubstituiertem Phenethenyl, un-, mono- oder disubstituiertem (Phenyl-imino)methylen, un-, mono- oder disubstituiertem (Phenylmethylen)imino und un-, mono- oder disubstituiertem Mono- und Diphenylamino, Piperidinyl, 3,5-Dimethylpiperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono-, di- oder trisubstituiertem 9,10-Dihydroacridinyl, un-, mono- oder disubstituiertem 1,2,3,4-Tetrahydrochinolinyl, un-, mono- oder disubstituiertem 2,3-Dihydro-1,4-benzoxazinyl un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus der Gruppe α ausgewählt sein können; oder wobei zwei direkt benachbarte Substituenten der Aryl- und Heteroaryl-Reste in a) und b) eine V-(CR₈R₉)ₚ-W-Gruppierung darstellen, wobei p = 1, 2 oder 3 ist, die Reste R₈ und R₉ jeweils unabhängig voneinander einen Substituenten, ausgewählt aus der Gruppe α, darstellen sowie V und W unabhängig voneinander aus den Gruppierungen -O-, -S-, -N(C₁ - C₆)-Alkyl, -NC₆H₅-, -CH₂-, -C(CH₃)₂- oder -C(C₆H₅)₂- ausgewählt sind;
oder zwei oder mehrere benachbarte -CR₈R₉-Gruppierungen sind Teil eines annellierten Benzol-Ringes, der un-, mono- oder disubstituiert sein kann, wobei die Substituenten wiederum aus der Gruppe α ausgewählt sein können;
oder V und/oder W stellt zusammen mit der jeweils benachbarten -CR₈R₉-Gruppierung einen annellierten Benzol-Ring dar, der un-, mono- oder disubstituiert sein kann, wobei die Substituenten wiederum aus der Gruppe α ausgewählt sein können.

In einer bevorzugten Ausführungsform der vorliegenden Erfindung ist der Rest R₃ in den vorstehenden Formel (I) und (II) aus einem Wasserstoffatom, einem (C₁-C₆)-Alkylrest, einem (C₃-C₇)-Cycloalkylrest oder einem Phenylrest ausgewählt.

Vorzugsweise ist in den Formeln (I) und (II) der Rest R₂ der diesbezügliche un-, mono- oder disubstituierte Phenyl-Rest.

Bevorzugte photochrome Naphthopyrane gemäß der vorliegenden Erfindung weisen die Formel (II) auf.

In der Formel (II) stehen X und Y besonders bevorzugt beide für O (Sauerstoff). In einer anderen Ausführungsform kann X für CH₂ und Y für O stehen oder Y für CH₂ und X für O stehen.

In einer weiter bevorzugten Ausführungsform der vorliegenden Erfindung weisen die photochromen Naphthopyrane die nachstehende Formel (III) auf. 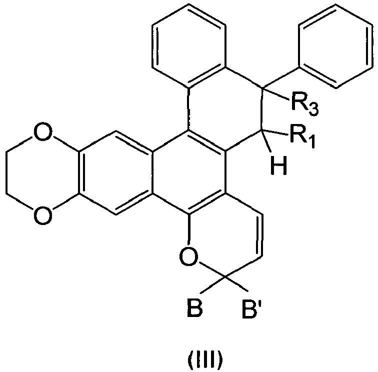
worin der Rest R₁ einen (C₁ - C₆)-Alkyl-Rest oder einen Phenyl-Rest darstellt;
der Rest R₃ Wasserstoff, einen (C₁ - C₆)-Alkyl-Rest oder einen Phenyl-Rest darstellt;
und die Reste B und B' wie vorstehend definiert sind.

Der Molekülstruktur der erfindungsgemäßen Verbindungen liegt eine Dihydrophenanthren-Untereinheit zugrunde (mit den Substituenten R₁ bis R₄ in den Formelbildern). An einem Benzolring dieser Dihydrophenanthren-Untereinheit ist sowohl ein weiterer Benzolring (mit den Substituenten bzw. der Substituentengruppe X-(CR₆R₇)ₘ-Y) bzw. ein 1,4-Benzodioxan-Ring (s. Formel (III) annelliert als auch eine photolabile Pyran-Einheit (mit den Substituenten B und B') gebunden. Letztere ist verantwortlich für den photochromen Charakter, da durch Anregung mit langwelligem UVA-Licht die Bindung zwischen dem Sauerstoff der Pyran-Einheit und dem Kohlenstoffatom mit den Substituenten B und B' reversibel gebrochen wird, wodurch ein farbiges Merocyanin-System entsteht.

Der vorliegenden Erfindung liegt der überraschende Befund zugrunde, dass die erfindungsgemäßen Verbindungen im Gegensatz zum nächstliegenden Stand der Technik (US 6,506,538) - eingebracht z.B. in eine Acrylatmonomer-Matrix mit UV-Initiator - unbeschadet eine durch starkes UV-Licht ausgelöste radikalische Polymerisation der Matrix überstehen. Diese hervorragende UV-Kompatibilität ist Folge der erfindungsgemäßen Einführung von mindestens zwei Substituenten (R₁ und R₂) - davon mindestens einer ein unsubstituierter oder substituierter Phenyl-Rest - in die Dihydrophenanthren-Untereinheit des Moleküls. Dadurch werden photochrome Farbstoffe mit interessanten spektralen Eigenschaften zugänglich, die eingebracht z.B. in eine Acrylat-Matrix eine überragende Stabilität sowohl hinsichtlich den Polymerisationsbedingungen als auch der Lebensdauer aufweisen.

Darüber hinaus weisen die erfindungsgemäßen Verbindungen bei geeigneter Substituentenwahl eine ausgeprägte Doppelabsorptionsbande der offenen Form im sichtbaren Wellenlängenbereich auf. Die eine Bande weist dabei ein Absorptionsmaximum von >500 nm auf, während das Maximum der zweiten Bande im kürzerwelligen sichtbaren Bereich (400-500 nm) liegt. Aufgrund letzterer Bande wird es mit der vorliegenden Erfindung möglich, in phototropen Farbstoffmischungen auf gelb- oder orangeeindunkelnde photochrome Farbstoffe zu verzichten. Dies ist einerseits wichtig für Polymersysteme, in denen diese gelb- und orangeeindunkelnden Farbstoffe - aufgrund ihrer anderen Molekülstruktur im Vergleich zu den längerwellig absorbierenden violett- und blaueindunkelnden Farbstoffen - eine ungenügende Lebensdauer aufweisen oder andere Nachteile mit sich bringen. Andererseits ist es mit einigen der erfindungsgemäßen photochromen Farbstoffen erstmals möglich, in Neutralfaben - d.h. in Grau- oder Brauntönen - eindunkelnde phototrope Gläser mit nur einem photochromen Farbstoff zu realisieren, die mittels UV-Härtung hergestellt werden. Damit entfällt der bisher notwendige mühsame Abstimmungsprozess zwischen den verschiedenen photochromen Farbstoffen einer Mischung hinsichtlich Aufhellungsgeschwindigkeit, Lebensdauer sowie spektralen Anregungseigenschaften, damit das phototrope Glas zu jedem Zeitpunkt des Eindunklungs- und Aufhellungscylus denselben Farbton aufweist.

Da die erfindungsgemäßen Verbindungen darüber hinaus hohe Augenklarheit (d.h. hohe Transmission im nicht angeregten Zustand) sowie sehr gute Lichtbeständigkeit aufweisen, sind sie hervorragend geeignet zum Einsatz in phototropen Gläsern.

Darüber hinaus sind die erfindungsgemäßen Verbindungen im nicht angeregten Zustand völlig farblos (d.h. ohne kosmetisch störenden Gelbton, da die Absorption der geschlossenen Form auf den UV-Bereich beschränkt ist) und weisen eine sehr gute Lebensdauer auf.
Fig. 1 zeigt ein entsprechendes Syntheseschema zur Herstellung der erfindungsgemäßen Verbindungen.
Fig. 2 zeigt die UV-Absorptionsspektren spezifischer erfindungsgemäßer Verbindung im Vergleich zum Stand der Technik.

Besonders bevorzugte Verbindungen der vorliegenden Erfindung sind:
3,3-Bis(4-methoxyphenyl)-6,7-ethylendioxy-13-phenyl-14-methyl-13,14-dihydronaphtho[2,1-d]naphtho[1,2-b]pyran ( = Erfindungsgemäße Verbindung 1)
3,3-Bis(4-methoxyphenyl)-6,7-ethylendioxy-13-phenyl-13,14-dimethyl-13,14-dihydronaphtho[2,1-d]naphtho[1,2-b]pyran (= Erfindungsgemäße Verbindung 2)
3,3-Bis(4-ethoxyphenyl)-6,7-ethylendioxy-13-phenyl-14-methyl-13,14-dihydronaphtho[2,1-d]naphtho[1,2-b]pyran
3,3-Bis(4-ethoxyphenyl)-6,7-ethylendioxy-13-phenyl-13,14-dimethyl-13,14-dihydronaphtho[2,1-d]naphtho[1,2-b]pyran
3-(4-Methoxyphenyl)-3-phenyl-6,7-ethylendioxy-13-phenyl-14-methyl-13,14-dihydronaphtho[2,1-d]naphtho[1,2-b]pyran
3-(4-Methoxyphenyl)-3-phenyl-6,7-ethylendioxy-13-phenyl-13,14-dimethyl-13,14-dihydronaphtho[2,1-d]naphtho[1,2-b]pyran (= Erfindungsgemäße Verbindung 3)
3-(4-Ethoxyphenyl)-3-phenyl-6,7-ethylendioxy-13-phenyl-14-methyl-13,14-dihydronaphtho[2,1-d]naphtho[1,2-b]pyran
3-(4-Ethoxyphenyl)-3-phenyl-6,7-ethylendioxy-13-phenyl-13,14-dimethyl-13,14-dihydronaphtho [2,1-d]naphtho[1,2-b]pyran
3-(4-Butoxyphenyl)-3-phenyl-6,7-ethylendioxy-13-phenyl-14-methyl-13,14-dihydronaphtho[2,1-d]naphtho[1,2-b]pyran
3-(4-Butoxyphenyl)-3-phenyl-6,7-ethylendioxy-13-phenyl-13,14-dimethyl-13,14-dihydronaphtho[2,1-d]naphtho[1,2-b]pyran.

In einer weiteren bevorzugten Ausführungsform sind die Reste B und B' in den vorstehenden Formel (I), (II) bzw. (III) unabhängig voneinander aus der Gruppe a), wie vorstehend definiert, ausgewählt. Besonders bevorzugt sind mono-, di- und trisubstituierte Aryl-Reste, wobei der Aryl-Rest ein Phenylrest ist.

Wenn zwei oder mehrere benachbarte -CR₆R₇-Gruppierungen in der Formel (II) Teil eines annellierten Benzol-Ringes, der un-, mono- oder disubstituiert sein kann, wobei die Substituenten wiederum aus der Gruppe α ausgewählt sein können, sind, liegt vorzugsweise folgende Struktureinheit vor:

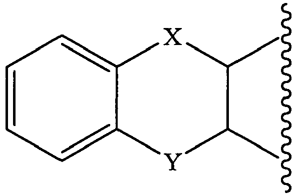

Wenn in der Formel (II) X und/oder Y zusammen mit der jeweils benachbarten -CR₆R₇-Gruppierung einen annellierten Benzol-Ring, der un-, mono- oder disubstituiert sein kann, wobei die Substituenten wiederum aus der Gruppe α ausgewählt sein können, darstellt, liegt vorzugsweise folgende Struktureinheit vor, wobei X vorzugsweise für O steht:

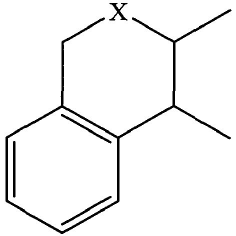

Die Substituenten der Gruppe χ, welche Stickstoffatome aufweisen bzw. Amingruppen tragen, sind über diese an den Phenyl-, Naphthyl- bzw. Phenanthrylrest der Gruppe a) gebunden.

Wenn bezüglich der Substituenten der Gruppe V-(CR₈R₉)ₚ-W-Gruppierung, welche an den Phenyl, Naphthyl- bzw. Phenanthrylrest der Gruppe a) für die Reste B bzw. B' gebunden sein können, zwei oder mehrere benachbarte Kohlenstoffatome dieser V-(CR₈R₉)ₚ-W-Gruppierung jeweils unabhängig voneinander Teil eines daran annellierten Benzo-Ringsystems sind, so bedeutet dies, dass dann die beiden Methylenkohlenstoffatome (-CH₂-CH₂-) Teil eines annellierten Ringsystems werden. Wenn beispielsweise zwei oder drei Benzoringe annelliert sind, so können beispielsweise hier dann folgende Struktureinheiten vorliegen, wie nachstehend angeführt.

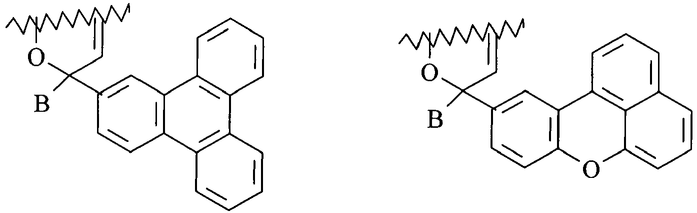

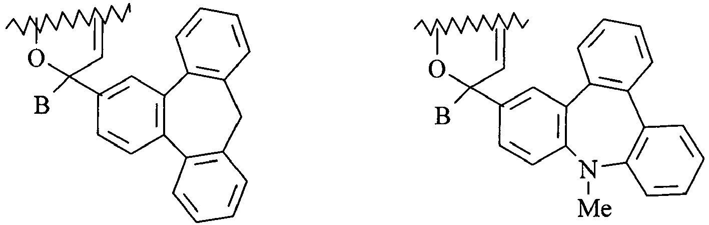

Selbstverständlich kann aber auch nur ein, über zwei benachbarte Kohlenstoffatome dieser V-(CR₈R₉)ₚ-W-Gruppierung annellierter Benzoring vorliegen.

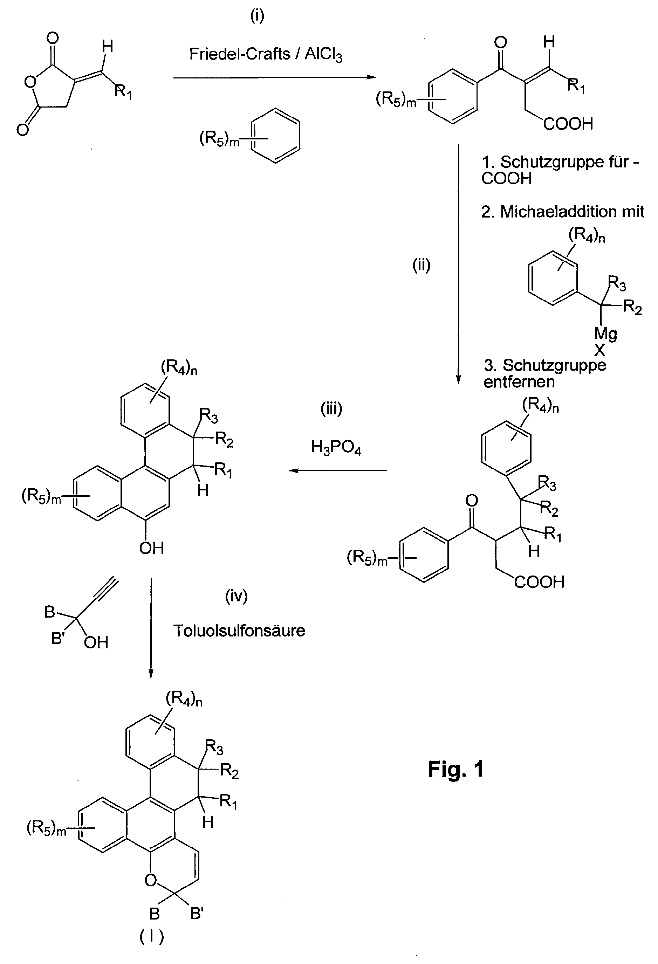

Zur Synthese der erfindungsgemäßen Verbindungen werden geeignet substituierte Methylidensuccinanhydride in einem ersten Schritt einer Friedel-Crafts-Reaktion mit geeignet substituierten Benzol-Derivaten unterworfen (Schritt (i)). Die -COOH Gruppe des daraus resultierenden Intermediats wird anschließend geschützt und dieses Intermediat einer Michael-Addition mit entsprechend substituierten benzylischen Grignardverbindungen unterworfen (Schritt (ii)). Nach Entfernung der Carbonsäure-Schutzgruppe werden *via* intramolekularer Cyclisierung mittels Phosphorsäure entsprechend substituierte Derivate gebildet (Schritt (iii)). Anschließend werden diese substituierten Derivate mit geeignet substituierten 2-Propin-1-ol-Derivaten gemäß Schritt (iv) zu den erfindungsgemäßen Verbindungen umgesetzt. Das vorstehende Syntheseschema ist in Figur 1 wiedergegeben.

Zur Messung der spektralen und photochromen Eigenschaften der erfindungsgemäßen Verbindungen wurden jeweils 1.5 Gew% des photochromen Farbstoffes zusammen mit einem handelsüblichen UV-Initiator für radikalische Polymerisationen in einer flüssig-viskosen Acrylatmonomer-Matrix gelöst. Mittels Spin-Coating oder Rakeln wurde eine dünne Schicht dieser viskosen Farbstofflösung auf einem transparenten Kunststoff-Substrat so aufgebracht, dass die Schichtdicke etwa 40 µm beträgt. Anschließend wurde die Schicht mittels UV-Licht gehärtet (polymerisiert).

Die photochromen Eigenschaften der so hergestellten Probekörper wurden anschließend gemäß DIN EN ISO 8980-3 ermittelt (Eindunklungsleistung bei 23°C zu definierten Belichtungsbedingungen).

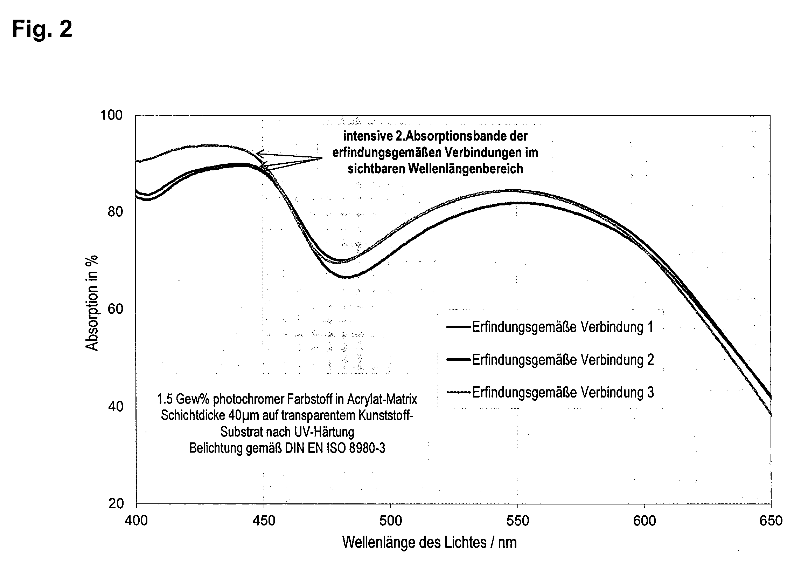

Die Absorptionsspektren beispielhaft ausgewählter, erfindungsgemäßer Verbindungen im angeregten Zustand sind in Figur 2 gezeigt. Die Ergebnisse der Messungen der Eindunklungsleistung nach DIN EN ISO 8980-3 sowie der Lebensdauerstabilität sind nachstehend in Tabelle 1 gezeigt.

Dabei weisen die in der Figur 2 aufgeführten, erfindungsgemäßen Verbindungen bezogen auf Formel (III) folgende Molekülstruktur auf:

| | R₁ | R₃ | B | B' |
|---|---|---|---|---|
| Erfindungsgemäße Verbindung 1 | Methyl | H | 4-Methoxyphenyl | 4-Methoxyphenyl |
| Erfindungsgemäße Verbindung 2 | Methyl | Methyl | 4-Methoxyphenyl | 4-Methoxyphenyl |
| Erfindungsgemäße Verbindung 3 | Methyl | Methyl | 4-Methoxyphenyl | Phenyl |

Die erfindungsgemäßen Verbindungen weisen eine ausgeprägte Doppelabsorptionsbande auf, wobei das kürzerwellige Absorptionsmaximum etwas stärker ausgeprägt ist als das längerwellige. Dadurch ergeben sich braune Farbtöne in der Eindunklung.

Die Tabelle 1 zeigt einen Vergleich der Eindunklungsleistungen (d.h. der Sättigungsabsorptionen aus Messungen gemäß DIN EN ISO 8980-3) im angeregten Zustand vor (d.h. direkt nach UV-Härtung) sowie nach Lebensdauertest. Zur Messung der Lebensdauerstabilität werden die Probekörper in einer Belichtungsapparatur 50h lang einer starken UV-Strahlung ausgesetzt (Simulation einer mehrjährigen Belastung durch Sonnenlicht).

**Tabelle 1: Photochrome Eigenschaften erfindungsgemäßer Verbindungen (An = Anisyl, d.h. der 4-Methoxyphenyl-Rest)**

| | |
|---|---|
| 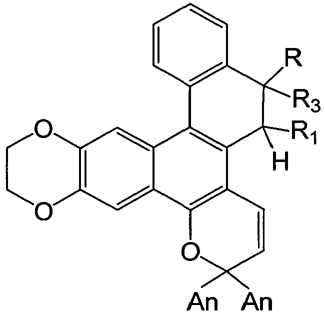 | 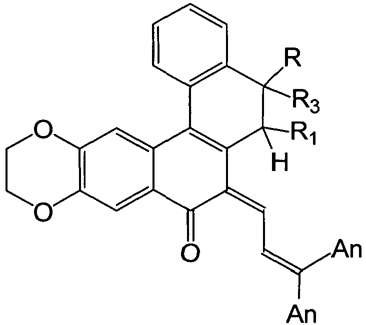 |
| nicht angeregt (farblos) | angeregt (farbig) |

Die nachstehende Tabelle 1 enthält zwei Verbindungen aus dem Stand der Technik (US 6,506,538) sowie zwei nicht erfindungsgemäße Vergleichsverbindungen mit R = Phenyl und R₁ = H.

Erfindungsgemäße Verbindungen in der Tabelle 1 umfassen nur Verbindungen mit R = Phenyl und zugleich R₁ = Methyl. R entspricht R₂ in den vorstehenden Formeln (I) und (II).

**Tabelle 1**

| | R | R₁ | R₃ | Sättigungsabsorption nach UV-Härtung | Sättigungsabsorption nach Lebensdauertest |
|---|---|---|---|---|---|
| Stand der Technik aus US 6,506,538 | H | H | H | 63% | 15% |
| Stand der Technik aus US 6,506,538 | H | Methyl | Ethyl | 65% | 47% |
| Vergleichsverbindung (nicht erfindungsgemäß) | Phenyl | H | H | 64% | 44% |
| Vergleichsverbindung (nicht erfindungsgemäß) | Phenyl | H | Phenyl | 65% | 49% |
| Erfindungsgemäße Verbindung 1 | Phenyl | Methyl | H | 77% | 73% |
| Erfindungsgemäße Verbindung 2 | Phenyl | Methyl | Methyl | 78% | 76% |

Wie aus der Tabelle 1 anschaulich hervorgeht, weisen nur die UV-gehärteten Probekörper mit den erfindungsgemäßen Verbindungen eine für kommerzielle UV-gehärtete phototrope Brillengläser ausreichende UV-Härtungs- und Lebensdauerstabilität auf, d.h. diese Probekörper zeigen einen nur sehr geringfügigen Verlust der Sättigungsabsorption nach Lebensdauertest.

Bei den Verbindungen aus dem Stand der Technik bzw. den nicht erfindungsgemäßen Vergleichsverbindungen ist zum einen bereits eine geringere Sättigungsabsorption im Vergleich zu den erfindungsgemäßen Verbindungen schon vor dem Lebesdauertest zu beobachten - diese Verbindungen zersetzen sich teilweise bereits bei der kurzzeitgen, aber starken Bestrahlung mit UV-Licht bei der UV-Härtung. Zum anderen weisen diese Verbindungen nach dem Lebensdauertest einen weiteren, drastischen Verlust an Sättigungsabsorption auf, der sie zum Einsatz in UV-gehärteten phototropen Brillengläsern ungeeignet macht.

Der Grund für die im Gegensatz zu den Verbindungen aus dem Stand der Technik drastisch erhöhte UV-Härtungs- und Lebensdauerstabilität liegt in der erfindungsgemäßen Kombination der Einführung eines Phenylsubstituenten R zusammen mit der Einführung eines weiteren Substiutenten R₁ am benachbarten Kohlenstoffatom (in Tabelle 1 eine Methylgruppe).

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Naphthopyran-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen und dergleichen eingesetzt werden. Ferner können die erfindungsgemäßen photochromen Naphthopyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Naphthopyrane durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Naphthopyrane beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

## Patentansprüche

1. Photochrome annellierte Naphthopyrane gemäß den allgemeinen Formeln (I) oder (II): 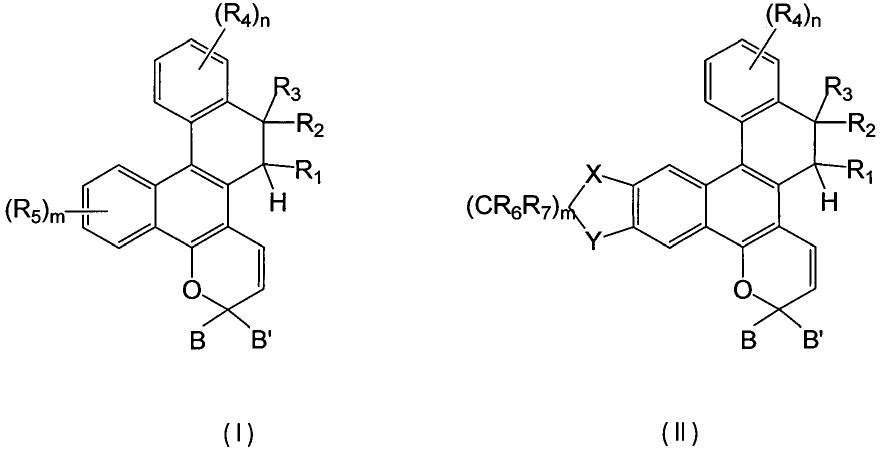
worin die Reste R₃, R₄ und R₅ jeweils unabhängig voneinander einen Substituenten darstellen, ausgewählt aus der Gruppe α, bestehend aus einem Wasserstoffatom, einem (C₁ - C₆)-Alkyl-Rest, einem (C₁ - C₆)-Thioalkyl-Rest, einem (C₃-C₇)-Cycloalkyl-Rest, der ein oder mehrere Heteroatome, wie beispielsweise O oder S, aufweisen kann, einem (C₁ - C₆)-Alkoxy-Rest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxy-Rest, wobei die Substituenten wiederum aus der Gruppe α ausgewählt sein können; wobei n, wenn nicht 0, eine ganze Zahl von 1 bis 4 darstellt;
oder zwei Reste R₄ bilden einen annellierten Benzol-Ring, der un-, mono- oder disubstituiert sein kann, wobei die Substituenten wiederum aus der Gruppe α ausgewählt sein können;
oder zwei Reste R₅ bilden einen annellierten Benzol-Ring, der un-, mono- oder disubstituiert sein kann, wobei die Substituenten wiederum aus der Gruppe α ausgewählt sein können;
und worin die Reste R₁ und R₂ jeweils unabhängig voneinander einen (C₁ - C₆)-Alkyl-Rest oder einen un-, mono- oder disubstituierten Phenyl-Rest darstellen, wobei die Substituenten wiederum aus der Gruppe α ausgewählt sein können; und mit der Maßgabe, dass zumindest einer der Reste R₁ oder R₂ einen un-, mono- oder disubstituierten Phenyl-Rest darstellt;
und worin X und Y unabhängig voneinander ausgewählt sind aus der Gruppe, bestehend aus -O-, -S- -N(C₁-C₆)-Alkyl, -NC₆H₅-, -CH₂-, -C(CH₃)₂- oder -C(C₆H₅)₂-;
und worin die Reste R₆ und R₇ jeweils unabhängig voneinander einen Substituenten darstellen, ausgewählt aus der Gruppe α;
m, wenn nicht 0, eine ganze Zahl von 1 bis 4 darstellt;
oder zwei oder mehrere benachbarte -CR₆R₇-Gruppierungen sind Teil eines annellierten Benzol-Ringes, der un-, mono- oder disubstituiert sein kann, wobei die Substituenten wiederum aus der Gruppe α ausgewählt sein können;
oder X und/oder Y stellt zusammen mit der jeweils benachbarten -CR₆R₇-Gruppierung einen annellierten Benzol-Ring dar, der un-, mono- oder disubstituiert sein kann, wobei die Substituenten wiederum aus der Gruppe α ausgewählt sein können;
und worin B und B' unabhängig voneinander aus einer der folgenden Gruppen a) oder b) ausgewählt ist, wobei
c) ein mono-, di- und trisubstituierter Aryl-Rest ist, wobei der Aryl-Rest Phenyl, Naphthyl oder Phenanthryl ist und
d) ein un-, mono- und disubstituierter Heteroaryl-Rest ist, wobei der Heteroaryl-Rest Pyridyl, Furanyl, Thienyl, Benzofuranyl, Benzothienyl, 1,2,3,4-Tetrahydrocarbazolyl und Julolidinyl ist;
wobei die Substituenten der Aryl- und Heteroaryl-Reste in a) und b) ausgewählt sind aus der Gruppe α oder der Gruppe χ, bestehend aus Amino, Mono-(C₁ - C₆)-alkylamino, Di-(C₁ - C₆)-alkylamino, am Phenylring un-, mono- oder disubstituiertem Phenethenyl, un-, mono- oder disubstituiertem (Phenyl-imino)methylen, un-, mono- oder disubstituiertem (Phenylmethylen)imino und un-, mono- oder disubstituiertem Mono- und Diphenylamino, Piperidinyl, 3,5-Dimethylpiperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono-, di- oder trisubstituiertem 9,10-Dihydroacridinyl, un-, mono- oder disubstituiertem 1,2,3,4-Tetrahydrochinolinyl, un-, mono- oder disubstituiertem 2,3-Dihydro-1,4-benzoxazinyl un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus der Gruppe α ausgewählt sein können;
oder wobei zwei direkt benachbarte Substituenten der Aryl- und Heteroaryl-Reste in a) und b) eine V-(CR₈R₉)ₚ-W-Gruppierung darstellen, wobei p = 1, 2 oder 3 ist, die Reste R₈ und R₉ jeweils unabhängig voneinander einen Substituenten, ausgewählt aus der Gruppe α, darstellen sowie V und W unabhängig voneinander aus den Gruppierungen -O-, -S-, -N(C₁ - C₆)-Alkyl, -NC₆H₅-, -CH₂-, -C(CH₃)₂- oder -C(C₆H₅)₂- ausgewählt sind;
oder zwei oder mehrere benachbarte -CR₈R₉-Gruppierungen sind Teil eines annellierten Benzol-Ringes, der un-, mono- oder disubstituiert sein kann, wobei die Substituenten wiederum aus der Gruppe α ausgewählt sein können;
oder V und/oder W stellt zusammen mit der jeweils benachbarten -CR₈R₉-Gruppierung einen annellierten Benzol-Ring dar, der un-, mono- oder disubstituiert sein kann, wobei die Substituenten wiederum aus der Gruppe α ausgewählt sein können.

2. Photochrome annellierte Naphthopyrane gemäß Anspruch 1, worin der Rest R₃ in den vorstehenden Formel (I) und (II) aus einem Wasserstoffatom, einem (C₁-C₆)-Alkylrest, einem (C₃-C₇)-Cycloalkylrest oder einem Phenylrest ausgewählt.

3. Photochrome annellierte Naphthopyrane gemäß Anspruch 1 oder 2, worin in den Formeln (I) und (II) der Rest R₂ der diesbezügliche un-, mono- oder disubstituierte Phenyl-Rest ist.

4. Photochrome annellierte Naphthopyrane gemäß einem der Ansprüche 1 bis 3, welche die Formel (II) aufweisen.

5. Photochrome annellierte Naphthopyrane gemäß einem der Ansprüche 1 bis 4, welche die nachstehende Formel (III) aufweisen: 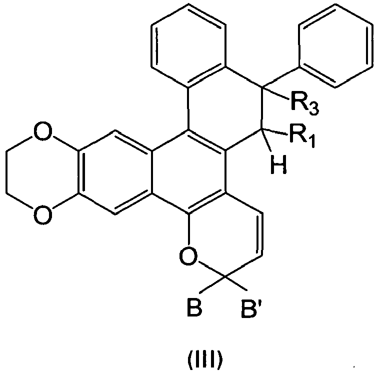
worin der Rest R₁ einen (C₁ - C₆)-Alkyl-Rest oder einen Phenyl-Rest darstellt;
der Rest R₃ Wasserstoff, einen (C₁ - C₆)-Alkyl-Rest oder einen Phenyl-Rest darstellt;
und die Reste B und B' wie vorstehend definiert sind.

6. Photochrome annellierte Naphthopyrane gemäß einem der Ansprüche 1 bis 5, welche sind:
3,3-Bis(4-methoxyphenyl)-6,7-ethylendioxy-13-phenyl-14-methyl-13,14-dihydronaphtho[2,1-d]naphtho[1,2-b]pyran
3,3-Bis(4-methoxyphenyl)-6,7-ethylendioxy-13-phenyl-13,14-dimethyl-13,14-dihydronaphtho[2,1-d]naphtho[1,2-b]pyran
3,3-Bis(4-ethoxyphenyl)-6,7-ethylendioxy-13-phenyl-14-methyl-13,14-dihydro-naphtho[2,1-d]naphtho[1,2-b]pyran
3,3-Bis(4-ethoxyphenyl)-6,7-ethylendioxy-13-phenyl-13,14-dimethyl-13,14-dihydronaphtho[2,1-d]naphtho[1,2-b]pyran
3-(4-Methoxyphenyl)-3-phenyl-6,7-ethylendioxy-13-phenyl-14-methyl-13,14-dihydronaphtho[2,1-d]naphtho[1,2-b]pyran
3-(4-Methoxyphenyl)-3-phenyl-6,7-ethylendioxy-13-phenyl-13,14-dimethyl-13,14-dihydronaphtho[2,1-d]naphtho[1,2-b]pyran
3-(4-Ethoxyphenyl)-3-phenyl-6,7-ethylendioxy-13-phenyl-14-methyl-13,14-dihydronaphtho[2,1-d]naphtho[1,2-b]pyran
3-(4-Ethoxyphenyl)-3-phenyl-6,7-ethylendioxy-13-phenyl-13,14-dimethyl-13,14-dihydronaphtho [2,1-d]naphtho[1,2-b]pyran
3-(4-Butoxyphenyl)-3-phenyl-6,7-ethylendioxy-13-phenyl-14-methyl-13,14-dihydronaphtho[2,1-d]naphtho[1,2-b]pyran
3-(4-Butoxyphenyl)-3-phenyl-6,7-ethylendioxy-13-phenyl-13,14-dimethyl-13,14-dihydronaphtho[2,1-d]naphtho[1,2-b]pyran.

7. Photochrome annellierte Naphthopyrane gemäß einem der Ansprüche 1 bis 6, wobei die Reste B und B' unabhängig voneinander aus der Gruppe a), wie vorstehend definiert, ausgewählt sind.

8. Verwendung der photochromen annellierten Naphthopyrane nach einem der Ansprüche 1 bis 7 in und auf Kunststoffmaterialien.

9. Verwendung nach Anspruch 8, wobei das Kunststoffmaterial eine ophthalmische Linse ist.

## Claims

1. Photochromic fused naphthopyranes according to the general formula (I) or (II): 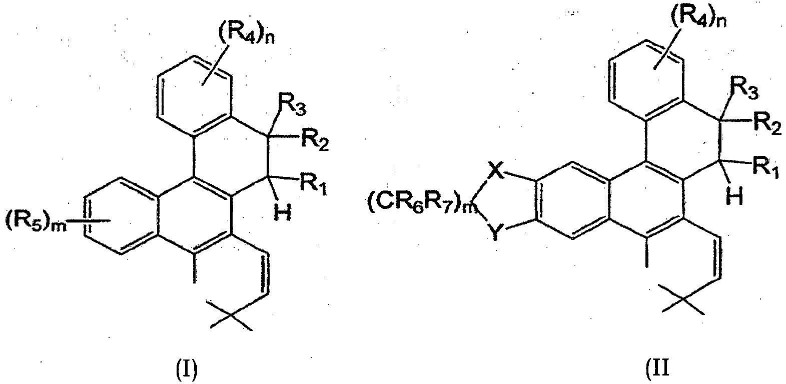
in which the radicals R₃, R₄ and R₅ represent respectively, independently of each other, a substituent selected from the group α, consisting of a hydrogen atom, a (C₁-C₆)-alkyl radical, a (C₁-C₆)-thioalkyl radical, a (C₃-C₇)-cycloalkyl radical which can have one or more heteroatoms, such as for example O or S, a (C₁-C₆)-alkoxy radical, a hydroxy group, a trifluoromethyl group, bromine, chlorine, fluorine, an un-, mono- or disubstituted phenyl-, phenoxy, benzyl-, benzyloxy, naphthyl- or naphthoxy radical, the substituents in turn being able to be selected from the group α; n, if not 0, representing an integer from 1 to 4;
or two radicals R₅ form a fused benzene ring, which can be un-, mono- or disubstituted, the substituents in turn being able to be selected from the group α;
or two radicals R₅ form a fused benzene ring, which can be un-, mono- or disubstituted, the substituents in turn being able to be selected from the group α;
and in which the radicals R₁ and R₂ represent respectively, independently of each other, a (C₁-C₆)-alkyl radical or an un-, mono- or disubstituted phenyl radical, the substituents in turn being able to be selected from the group α; and with the proviso that at least one of the radicals R₁ or R₂ represents an un-, mono- or disubstituted phenyl radical;
and in which X and Y, independently of each other, are selected from the group consisting of -O-, -S-, -N(C₁-C₆)-alkyl, -NC₆H₅-, -CH₂-, -C(CH₃)₂- or -C(C₆H₅)₂-;
and in which the radicals R₆ and R₇ represent respectively, independently of each other, a substituent selected from the group α;
m, if not 0, represents an integer from 1 to 4;
or two or more adjacent -CR₆R₇- groupings are part of a fused benzene ring, which can be un-, mono- or disubstituted, the substituents in turn being able to be selected from the group α; or X and/or Y represent, together with the respectively adjacent -CR₆R₇- grouping, a fused benzene ring which can be un-, mono- or disubstituted, the substituents in turn being able to be selected from the group α;
and in which B and B' are selected, independently of each other, from one of the following groups a) or b),
c) being a mono-, di- or trisubstituted aryl radical, the aryl radical being phenyl, naphthyl or phenanthryl and
d) being an un-, mono- and disubstituted heteroaryl radical, the heteroaryl radical being pyridyl, furanyl, thienyl, benzofuranyl, benzothienyl, 1,2,3,4-tetrahydrocarbazolyl and julodinyl;
the substituents of the aryl- and heteroaryl radicals in a) and b) being selected from the group α or the group χ consisting of amino, mono-(C₁-C₆)-alkyamino, di(C₁-C₆)-alkylamino, phenethenyl which is un-, mono- or disubstituted on the phenyl ring, un-, mono- or disubstituted (phenylimino)methylene, un-, mono- oder disubstituiertem (phenylmethylene)imino and un-, mono- or disubstituted mono- and diphenylamino, piperidinyl, 3,5-dimethylpiperidinyl, N-substituted piperazinyl, pyrrolidinyl, imidazolidinyl, pyrazolidinyl, indolinyl, morpholinyl, 2,6-dimethylmorpholinyl, thiomorpholinyl, azacycloheptyl, azacyclooctyl, un-, mono- or disubstituted phenothiazinyl, un-, mono- or disubstituted phenoxazinyl, un-, mono-, di- or trisubstituted 9,10-dihydroacridinyl, un-, mono- or disubstituted 1,2,3,4-tetrahydroquinolinyl, un-, mono- or disubstituted 2,3-dihydro-1,4-benzoxazinyl, un-, mono- or disubstituted 1,2,3,4-tetrahydroisoquinolinyl, un-, mono- or disubstituted phenazinyl, un-, mono- or disubstituted carbazolyl, un-, mono- or disubstituted 1,2,3,4-tetrahydrocarbazolyl and un-, mono- or disubstituted 10,11-dihydrodibenz[b,f]azepinyl, the substituent(s), independently of each other, being able in turn to be selected from the group α;
or two directly adjacent substituents of the aryl- and heteroaryl radicals in a) and b) representing a V-(CR₈R₉)ₚ-W- grouping, p being = 1, 2 or 3,
the radicals R₈ and R₉ representing respectively, independently of each other, a substituent selected from the group α; and also V and W, independently of each other, being selected from the groupings -O-, - S-, -N(C₁-C₆)-alkyl, -NC₆H₅-, -CH₂-, -C(CH₃)₂- or -C(C₆H₅)₂-;
or two or more adjacent -(CR₈R₉)-groupings being part of a fused benzene ring, which can be un-, mono- or disubstituted, the substituents in turn being able to be selected from the group α; or V and/or W representing, together with the respectively adjacent -(CR₈R₉)ₚ- grouping, a fused benzene ring, which can be un-, mono- or disubstituted, the substituents in turn being able to be selected from the group α.

2. Photochromic fused naphthopyranes according to claim 1, in which the radical R₃ in the previous formulae (I) and (II) is selected from a hydrogen atom, a (C₁-C₆)-alkyl radical, a (C₃-C₇)-cycloalkyl radical or a phenyl radical.

3. Photochromic fused naphthopyranes according to claim 1 or 2, in which the radical R₂ in the formulae (I) and (II) is the phenyl radical which is un-, mono- or disubstituted relative thereto.

4. Photochromic fused naphthopyranes according to one of the claims 1 to 3, which have the formula (II).

5. Photochromic fused naphthopyranes according to one of the claims 1 to 4, which have the following formula (III): 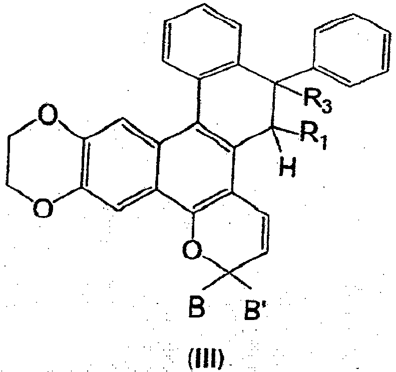
in which the radical R₁ represents a (C₁-C₆)-alkyl radical or a phenyl radical;
the radical R₃ represents hydrogen, a (C₁-C₆)-alkyl radical or a phenyl radical;
and the radicals B and B' are defined as above.

6. Photochromic fused naphthopyranes according to one of the claims 1 to 5, which are:
3,3-bis(4-methoxyphenyl)-6,7-ethlenedioxy-13-phenyl-14-methyl-13,14-dihydronaphtho [2,1-d]naphtho[1,2-b]pyrane
3,3-bis(4-methoxyphenyl)-6,7-ethylenedioxy-13-phenyl-13,14-dimethyl-13,14-dihydronaphtho[2,1-d]naphtho[1,2-b]pyrane
3,3-bis(4-ethoxyphenyl)-6,7-ethylenedioxy-13-phenyl-14-methyl-13,14-dihydronaphtho[2,1-d]naphtho[1,2-b]pyrane
3,3-bis(4-ethoxyphenyl)-6,7-ethylenedioxy-13-phenyl-13,14-dimethyl-13,14-dihydronaphtho[2,1-d]naphtho[1,2-b]pyrane
3-(4-methoxyphenyl)-3-phenyl-6,7-ethylenedioxy-13-phenyl-14-methyl-13,14-dihydronaphtho[2,1-d]naphtho[1,2-b]pyrane
3-(4-methoxyphenyl)-3-phenyl-6,7-ethylenedioxy-13-phenyl-13, 14-dimethyl-13,14-dihydronaphtho[2,1-d]naphtho[1,2-b]pyrane
3-(4-ethoxyphenyl)-3-phenyl-6,7-ethylenedioxy-13-phenyl-14-methyl-13,14-dihydronaphtho[2,1-d]naphtho[1,2-b]pyrane
3-(4-ethoxyphenyl)-3-phenyl-6,7-ethylenedioxy-13-phenyl-13,14-dimethyl-13,14-dihydronaphtho[2,1-d]naphtho[1,2-b]pyrane
3-(4-butoxyphenyl)-3-phenyl-6,7-ethylenedioxy-13-phenyl-14-methyl-13,14-dihydronaphtho[2,1-d]naphtho[1,2-b]pyrane
3-(4-butoxyphenyl)-3-phenyl-6,7-ethylenedioxy-13-phenyl-13, 14-dimethyl-13,14-dihydronaphtho[2,1-d]naphtho[1,2-b]pyrane.

7. Photochromic fused naphthopyranes according to one of the claims 1 to 6, the radicals B and B', independently of each other, being selected from the group α), as defined above.

8. Use of the photochromic fused naphthopyranes according to one of the claims 1 to 7 in and on plastic materials.

9. Use according to claim 8, the plastic material being an ophthalmic lens.

## Revendications

1. Naphtopyranes photochromes annelés selon les formules générales (I) ou (II) : 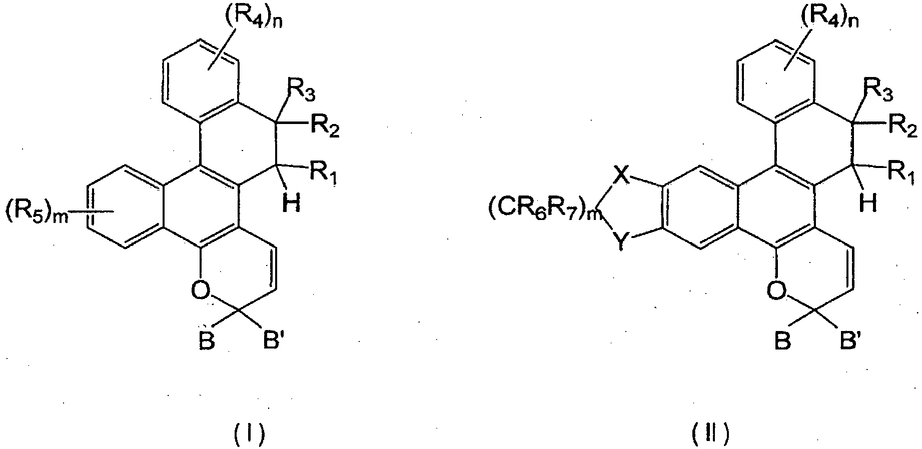
dans lesquelles les radicaux R₃, R₄ et R₅ représentent respectivement, indépendamment les uns des autres, un substituant choisi dans le groupe α, constitué d'un atome d'hydrogène, d'un radical alkyle (en C₁ à C₆), d'un radical thioalkyle (en C₁ à C₆), d'un radical cycloalkyle (en C₃ à C₇) qui peut présenter un ou plusieurs hétéroatomes, comme par exemple O ou S, un radical alcoxy (en C₁ à C₆), un groupe hydroxy, un groupe trifluorométhyle, un atome de brome, de chlore, de fluor, un radical phényle, phénoxy, benzyle, naphtyle ou naphtoxy non substitué, monosubstitué ou disubstitué, où les substituants peuvent eux-mêmes être choisis dans le groupe α ; où n, s'il n'est pas 0, représente un nombre entier de 1 à 4 ;
ou deux radicaux R₄ forment un cycle benzène annelé qui peut être non substitué, monosubstitué ou disubstitué, où les substituants peuvent eux-mêmes être choisis dans le groupe α ;
ou deux radicaux forment un cycle benzène annelé qui peut être non substitué, monosubstitué ou disubstitué, où les substituants peuvent eux-mêmes être choisis dans le groupe α ;
et dans lesquelles les radicaux R₁ et R₂ représentent respectivement indépendamment, un radical alkyle (en C₁ à C₆) ou un radical phényle non substitué, monosubstitué ou disubstitué, où les substituants peuvent eux-mêmes être choisis dans le groupe α ; et à condition qu'au moins l'un des radicaux R₁ ou R₂ représente un radical phényle non substitué, monosubstitué ou disubstitué ;
et dans lesquelles X et Y sont choisis indépendamment l'un de l'autre dans le groupe constitué de -O-, -S-, -N-alkyle (en C₁ à C₆), -NC₆H₅-, -CH₂-, -C(CH₃)₂- ou -C(C₆H₅)₂ ;
et dans lesquelles les radicaux et R₇ respectivement, représentent indépendamment l'un de l'autre, un substituant choisi dans le groupe α ;
m, s'il n'est pas 0, représente un nombre entier de 1 à 4 ;
ou deux ou plusieurs groupes -CR₆R₇- voisins font partie d'un cycle benzène annelé qui peut être non substitué, monosubstitué ou disubstitué, où les substituants peuvent eux-mêmes être choisis dans le groupe α ;
ou X et/ou Y représentent conjointement avec le groupe -CR₆R₇- voisin respectif, un cycle benzène annelé qui peut être non substitué, monosubstitué ou disubstitué, où les substituants peuvent eux-mêmes être choisis dans le groupe α ;
et dans lesquelles B et B' sont choisis indépendamment les uns des autres dans l'un des groupes a) ou b) suivants, où
c) est un radical aryle, monosubstitué, disubstitué et trisubstitué, où le radical aryle est un groupe phényle, naphtyle ou phénanthryle et
d) un radical hétéroaryle non substitué, monosubstitué et disubstitué, où le radical hétéroaryle est un groupe pyridyle, furanyle, thiényle, benzofuranyle, benzothiényle, 1,2,3,4-tétrahydrocarbazolyle et julolidinyle ;
où les substituants des radicaux aryle et hétéroaryle dans a) et b) sont choisis dans le groupe α ou le groupe χ, constitué des groupes amino, mono-alkylamino (en C₁ à C₆), di-alkylamino (en C₁ à C₆), phénéthényle non substitué, monosubstitué ou disubstitué sur le cycle phényle, (phényl-imino)méthylène non substitué, monosubstitué ou disubstitué, (phénylméthylène)imino non substitué, monosubstitué ou disubstitué et mono- et diphénylamino non substitué, monosubstitué ou disubstitué, pipéridinyle, 3,5-diméthylpipéridinyle, pipérazinyle N-substitué, pyrrolidinyle, imidazolidinyle, pyrazolidinyle, indolinyle, morpholinyle, 2,6-diméthylmorpholinyle, thiomorpholinyle, azacycloheptyle, azacyclo-octyle, phénothiazinyle non substitué, monosubstitué ou disubstitué, phénoxazinyle non substitué, monosubstitué ou disubstitué, 9,10-dihydroacridinyle non substitué, monosubstitué ou disubstitué, 1,2,3,4-tétrahydroquinolinyle non substitué, monosubstitué ou disubstitué, 2,3-dihydro-1,4-benzoxazinyle non substitué, monosubstitué ou disubstitué, 1,2,3,4-tétrahydro-isoquinolinyle non substitué, monosubstitué ou disubstitué, phénazinyle non substitué, monosubstitué ou disubstitué, carbazolyle non substitué, monosubstitué ou disubstitué, 1,2,3,4-tétrahydrocarbazolyle non substitué, monosubstitué ou disubstitué et 10,11-dihydrodibenz[b,f]azépinyle non substitué, monosubstitué ou disubstitué, où le ou les substituants peuvent être choisis eux-mêmes indépendamment les uns des autres dans le groupe α ;
ou où deux substituants directement voisins des radicaux aryle et hétéroaryle dans a) et b) représentent un groupe V- (CR₈R₉)ₚ-W, où p = 1, 2 ou 3, les radicaux R₈ et R₉ représentent respectivement, indépendamment l'un de l'autre, un substituant choisi dans le groupe α, et V et W sont choisis, indépendamment l'un de l'autre, dans les groupes -O-, -S-, -N-alkyle (en C₁ à C₆), -NC₆H₅-, -CH₂-, -C(CH₃)₂- ou -C(C₆H₅)₂- ;
ou deux ou plusieurs groupes -CR₈R₉- voisins font partie d'un cycle benzène annelé qui peut être non substitué, monosubstitué ou disubstitué ; où les substituants peuvent eux-mêmes être choisis dans le groupe α ;
ou V et/ou W représentent conjointement avec le groupe -CR₈R₉- voisin respectif, un cycle benzène annelé qui peut être non substitué, monosubstitué ou disubstitué, où les substituants peuvent eux-mêmes être choisis dans le groupe α.

2. Naphtopyranes photochromes annelés selon la revendication 1, dans lesquels le radical R₃ dans les formules (I) et (II) précédentes est choisi parmi un atome d'hydrogène, un radical alkyle (en C₁ à C₆), un radical cycloalkyle (en C₃ à C₇) ou un radical phényle.

3. Naphtopyranes photochromes annelés selon la revendication 1 ou 2, dans lesquels dans les formules (I) et (II), le radical R₂ est le radical phényle non substitué, monosubstitué ou disubstitué à cet égard.

4. Naphtopyranes photochromes annelés selon l'une des revendications 1 à 3, qui présentent la formule (II).

5. Naphtopyranes photochromes annelés selon l'une des revendications 1 à 4, qui présentent la formule (III) suivante : 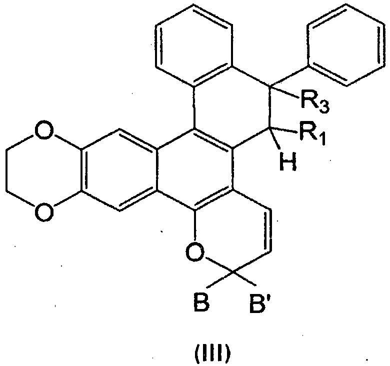
dans laquelle la radical R₁ représente un radical alkyle (en C₁ à C₆) ou un radical phényle ;
le radical R₃ représente un atome d'hydrogène, un radical alkyle (en C₁ à C₆) ou un radical phényle ;
et les radicaux B et B' sont tels que définis précédemment.

6. Naphtopyranes photochromes annelés selon l'une des revendications 1 à 5, qui sont
le 3,3-bis(4-méthoxyphényl)-6,7-éthylènedioxy-13-phényl-14-méthyl-13,14-dihydro-naphto[2,1-d]naphtol[1,2-b]pyrane
le 3,3-bis(4-méthoxyphényl)-6,7-éthylènedioxy-13-phényl-13,14-diméthyl-13,14-dihydro-naphto[2,1-d]naphtol[1,2-b]pyrane
le 3,3-bis(4-éthoxyphényl)-6,7-éthylènedioxy-13-phényl-14-méthyl-13,14-dihydro-naphto[2,1-d]naphtol[1,2-b]pyrane
le 3,3-bis(4-éthoxyphényl)-6,7-éthylènedioxy-13-phényl-13,14-diméthyl-13,14-dihydro-naphto[2,1-d]naphtol[1,2-b]pyrane
le 3-(4-méthoxyphényl)-3-phényl-6,7-éthylènedioxy-13-phényl-14-méthyl-13,14-dihydronaphto[2,1-d]naphtol[1,2-b]pyrane
le 3-(4-méthoxyphényl)-3-phényl-6,7-éthylènedioxy-13-phényl-13,14-diméthyl-13,14-dihydronaphto[2,1-d]naphtol[1,2-b]pyrane
le 3-(4-éthoxyphényl)-3-phényl-6,7-éthylènedioxy-13-phényl-14-méthyl-13,14-dihydronaphto[2,1-d]naphtol[1,2-b]pyrane
le 3-(4-éthoxyphényl)-3-phényl-6,7-éthylènedioxy-13-phényl-13,14-diméthyl-13,14-dihydronaphto[2,1-d]naphtol[1,2-b]pyrane
le 3-(4-butoxyphényl)-3-phényl-6,7-éthylènedioxy-13-phényl-14-méthyl-13,14-dihydronaphto[2,1-d]naphtol[1,2-b]pyrane
le 3-(4-butoxyphényl)-3-phényl-6,7-éthylènedioxy-13-phényl-13,14-diméthyl-13,14-dihydronaphto[2,1-d]naphtol[1,2-b]pyrane.

7. Naphtopyranes photochromes annelés selon l'une des revendications 1 à 6, dans lesquels les radicaux B et B' sont choisis indépendamment l'un de l'autre dans le groupe a) tel que défini précédemment.

8. Utilisation des naphtopyranes photochromes annelés selon l'une des revendication 1 à 7, dans et sur des matériaux en plastique.

9. Utilisation selon la revendication 8, dans laquelle le matériau en plastique est une lentille ophtalmique.
